# EUROPÄISCHE PATENTSCHRIFT

(11) **EP 2 665 810 B1**
(45) Veröffentlichungstag und Bekanntmachung des Hinweises auf die Patenterteilung: **12.10.2016**
(21) Anmeldenummer: 12700553.6
(22) Anmeldetag: 11.01.2012
(51) Int. Cl.: C12N 5/00

(54) **VERFAHREN ZUR IMMOBILISIERUNG UND AUFBEREITUNG FUNKTIONELLER MULTIKOMPONENTENSTRUKTUREN DER EXTRAZELLULÄREN MATRIX**
METHOD OF IMMOBILIZING AND PROCESSING FUNCTIONAL MULTICOMPONENT STRUCTURES OF THE EXTRACELLULAR MATRIX
PROCÉDÉ POUR L'IMMOBILISATION ET LA PRÉPARATION DE STRUCTURES DE MULTICOMPOSANTS FONCTIONNELLES DE MATRICE EXTRACELLULAIRE

(30) Priorität: 18.01.2011 DE 102011002839
(43) Veröffentlichungstag der Anmeldung: 27.11.2013
(73) Patentinhaber: Leibniz-Institut für Polymerforschung Dresden e.V., 01069 Dresden (DE)
(72) Erfinder: PREWITZ, Marina, 01099 Dresden (DE); FREUDENBERG, Uwe, 01328 Dresden (DE); WERNER, Carsten, 01069 Dresden (DE)
(74) Vertreter: Sperling, Thomas
(86) Internationale Anmeldenummer: PCT/EP2012/050356
(87) Internationale Veröffentlichungsnummer: WO 2012/098037

(56) Entgegenhaltungen:
- DE-A1-102009 002 577
- HERKLOTZ M ET AL: "The impact of primary and secondary ligand coupling on extracellular matrix characteristics and formation of endothelial capillaries", BIOMATERIALS, ELSEVIER SCIENCE PUBLISHERS BV., BARKING, GB, Bd. 30, Nr. 1, 1. Januar 2009 (2009-01-01) , Seiten 35-44, XP025561258, ISSN: 0142-9612, DOI: 10.1016/J.BIOMATERIALS.2008.09.023 [gefunden am 2008-10-05]
- MARTIN L DECARIS ET AL: "Design of Experiments Approach to Engineer Cell-Secreted Matrices for Directing Osteogenic Differentiation", ANNALS OF BIOMEDICAL ENGINEERING, KLUWER ACADEMIC PUBLISHERS-PLENUM PUBLISHERS, NE, Bd. 39, Nr. 4, 1. Dezember 2010 (2010-12-01), Seiten 1174-1185, XP019892615, ISSN: 1573-9686, DOI: 10.1007/S10439-010-0217-X

## Beschreibung

Die Erfindung betrifft ein Verfahren zur Immobilisierung und Aufbereitung funktioneller Multikomponentenstrukturen der extrazellulären Matrix.

Die *in vitro* Kultivierung von Zellen erfordert spezielle Methoden und Prozeduren, um Bedingungen zu schaffen, die denen im lebenden Organismus möglichst stark ähneln. Hierfür ist neben der Einstellung der Temperatur, der Gaszusammensetzung und der Komponenten der Nährlösung auch die Wahl der die Zelle umgebenden Materialien entscheidend. Gegenwärtig kommen in diesem Bereich unterschiedliche Kunststoffträger in Form von Einmalartikeln zum Einsatz, die als sogenannte TCP (= Tissue Culture Plastic) meist aus Polystyren bestehen. Die Zellen werden dabei direkt auf den Kunststoffoberflächen kultiviert. Die natürliche Zellumgebung, die sogenannte extrazelluläre Matrix, wird im Gegensatz dazu durch ein hochkomplexes Netzwerk aus supramolekularen Strukturen von Molekülen, wie Kollagen, Elastin, Proteoglykanen und Glykoproteinen, gebildet. Diese extrazelluläre Matrix reguliert und beeinflusst in ihrer Zusammensetzung die normale Funktion von Gewebearchitektur, Mikromilieu und Zellregulation. Wie aus der Druckschrift Lutolf, M.P. & Hubbell, J.A. "Synthetic biomaterials as instructive extracellular microenvironments for morphogenesis in tissue engineering", Nature biotechnology 23, 47-55 (2005**),** bekannt ist, nimmt dieses Mikromilieu der Zelle einen entscheidenden Einfluss auf Zellschicksalsentscheidungen, wie zum Beispiel Wachstum und Differenzierung der Zellen, und wird somit als einer der Schlüsselfaktoren bei der Untersuchung von Entwicklung und Regulation derartiger biologischer Systeme betrachtet. Um dieses komplexe Milieu und dessen Einfluss auf die Zelle zu untersuchen, werden gegenwärtig in der Forschung und auch teilweise in kommerziellen Produkten *in vitro* unterschiedliche Bestandteile der extrazellulären Matrix künstlich aufbereitet und in Form von einfachen Beschichtungen als Zellkulturträger zur Verfügung gestellt. Fundstellen für entsprechenden Stand der Technik sind die Druckschriften
- Bio-One, G. CellCoat - Protein Coated Cell Culture Vessels. http://www.greinerbioone.com/en/germany/articleslcatalogue/articl e-groups/23-1/ (2010),
- Millipore 24-well Plate with Collagen I coating. http://www.millipore.com/catalogue/item/picl24p05 (2010**),** sowie
- Millipore 6-well Plate with Fibronectin Coating. http://www.millipore.com/catalogue/item/pifb06p05 (2010**).**

Gegenwärtig werden hierfür einzelne Komponenten, beispielsweise Kollagen I oder Fibronektin, oder Kombinationen aus mehreren von natürlichen Quellen isolierten Bestandteilen der extrazellulären Matrix verwendet, um TCP-Oberflächen zu funktionalisieren, wobei die meisten Ansätze adsorptiv, nicht kovalent gebundene Biomoleküle verwenden. Funktionalisierungen mit Einzelkomponenten ermöglichen jedoch nur die Untersuchung einzelner Parameter der extrazellulären Matrix; die Eigenschaften sind noch immer weit von der *in vivo* Situation entfernt. Speziell die Netzwerkcharakteristik der extrazellulären Matrix, welche auch die Kultivierung der Zellen in komplexen 3D-Strukturen erlaubt, sowie die Fähigkeit, Signalmoleküle wie Cytokine oder Chemokine reversibel zu binden, sind aufgrund der Nutzung einzelner Bestandteile der extrazellulären Matrix erheblich eingeschränkt.

Ein weiterer Anwendungsnachteil besteht in der gegenwärtig meist nur adsorptiven Kopplung der Komponenten der extrazellulären Matrix auf den Substraten, wodurch keine Delaminationsstabilität gewährleistet wird. In Zellkulturanwendungen sind jedoch häufige Wasch- beziehungsweise Austauschschritte, zum Beispiel Mediumwechsel, Waschschritte vor der Fixierung und Färbung der Zellen, notwendig, bei denen bedingt durch den Flüssigkeitsaustausch Scherbelastungen auf die funktionellen Beschichtungen wirken. Die meisten derzeitig etablierten funktionellen Beschichtungen haben mitunter massive Stabilitätsprobleme.

Aus HERKLOTZ, M. et al: "The impact of primary and secondary ligand coupling on extracellular matrix characteristics and formation of endothelial capillaries", Biomaterials 30, (2009), Seiten 35 bis 44, sind Zellkulturträger mit aminosilanisierten Silikon-Mikrostrukturen der Grundsubstratoberfläche bekannt, an die Maleinsäureanhydrid-Copolymere kovalent gekoppelt sind. An die Maleinsäureanhydrid-Copolymere sind wiederum Proteine (Fibronektin) gekoppelt, die zunächst die äußere Beschichtung des Zellkulturträgers bilden, auf der dann Zellen platziert werden, die extrazelluläre Matrix (EZM) abscheiden. Die Zellschichten werden ganz vorsichtig in phosphatgepufferter Salzlösung (PBS) gewaschen und danach entweder durch Nutzung von Paraformaldehyd oder DTSSP (3,3'-Dithiobis-(sulfosuccinimidylpropionate) chemisch fixiert. Durch die chemische Fixierung werden freie Lysingruppen der Zelloberfläche und auch der darunterliegenden extrazellulären Matrix durch eine kovalente Reaktion entweder mittels der Aldehydgruppen am Paraformaldehyd oder des NHS-Esters am DTSSP umgesetzt. Danach sind die Zellen fixiert, das heißt chemisch kovalent vernetzt und auch kovalent an die extrazelluläre Matrix (EZM) gebunden. Anschließend werden die Zellwände durch Nutzung von Triton X-100 permeabilisiert, das heißt durchlässig für Antikörper für die anschließende Färbung gemacht oder es werden nichtfixierte Zellbestandteile durch Spülen mit Natriumdodecylsulfat (SDS) entfernt.

Die DE 10 2009 002 577 A1 offenbart Zellkulturträger, dessen Grundsubstrat mit Mikrokavitäten versehen ist. Eine funktionale Beschichtung des Grundsubstrats kann das Ergebnis einer kovalenten Bindung von Poly(ethylen-alt-Maleinsäureanhydrid) (PEMA) an die Grundsubstratoberfläche, zum Beispiel an aminosilanisierte Silikon-Mikrostrukturen, sein. Dieses Grundsubstrat erhält darüber hinaus eine oder mehrere funktionelle Beschichtungen von Polymeren, das heißt, dass die Beschichtungspolymere funktionelle Gruppen für die Kopplung mit weiteren Beschichtungspolymeren aufweisen. Als weitere Beschichtung ist gemäß der DE 10 2009 002 577 A1 unter anderem auch die Kopplung einer oder mehrerer verschiedener zelladhäsionsvermittelnder, in der extrazellulären Matrix (ECM) der Zell-Mikroumgebung im Knochenmark vorkommender Komponenten vorgesehen. Anschließend werden die Zellen an den beschichteten Zellkulturträgern entwickelt, wobei ein Teil der Zellen durch Adhäsion in den Mikrokavitäten am Zellkulturträger anhaftet.

Bei Decaris, M. et al.: "Design of Experiments Approach to Engineer Cell-Secreted Matrices for Directing Osteogenic Differentiation", Annals of Biomedical Engineering, Vol. 39, No. 4, 1 December 2010, Seiten 1174 bis 1185, ist eine Dezellularisierung von Zellschichten beschrieben, die mit einer Dezellularisierungslösung aus 0,1 % Triton-x-100 und 20 mM Ammoniumhydroxid in PBS erfolgt.

Die Aufgabe der Erfindung besteht in der Bereitstellung funktioneller Beschichtungen auf Zellkulturträgern, die der natürlichen Zellumgebung weitgehend entsprechen und delaminationsstabil gegenüber mechanischen Belastungen, insbesondere Scherbelastungen beim Flüssigkeitsaustausch und bei Waschschritten, sind.

Die Lösung der Aufgabe der Erfindung besteht in einem Verfahren zur Immobilisierung und Aufbereitung funktioneller Multikomponentenstrukturen der extrazellulären Matrix (EZM), umfassend die aufeinander folgenden Verfahrensschritte:
a) kovalente Bindung einer Haftvermittlerschicht aus Carbonsäuregruppen tragenden Polymeren, einschließlich Maleinsäureanhydrid-Copolymere und Polyacrylate, auf Zellkulturträgern;
b) Kultivierung von mesenchymalen Stammzellen auf der Haftvermittlerschicht und somit Immobilisierung der von den Zellen sezernierten extrazellulären Matrix (EZM) durch Abscheidung und Anbindung an die Haftvermittlerschicht; wobei die Haftvermittlerschicht der Stabilisierung der von den Zellen sezernierten extrazellulären Matrix (EZM) dient, und
c) Anwendung eines Dezellularisierungsprotokolls, um matrixsezernierende Zellen von der Oberfläche zu entfernen und gleichzeitig die Struktur und Funktionalität der darunterliegenden, mit dem Haftvermittler verbundenen immobilisierten extrazellulären Matrix (EZM) zu erhalten; und
d) eine Funktionalisierung der nach den Verfahrensschritten a) bis c) aufbereiteten extrazellulären Matrix (EZM) mit Signalmolekülen.

Im Rahmen des erfindungsgemäßen Verfahrens werden natürliche extrazelluläre Matrices dezellularisiert und die dadurch gewonnenen Netzwerke bedarfsgerecht als weitgehend naturidentische Zellumgebung in Zellkulturanwendungen eingesetzt. Der Fokus liegt dabei auf einer weitgehenden Erhaltung der ursprünglichen Funktionalität der Strukturen der extrazellulären Matrix, besonders in Bezug auf Adhäsivität und der Beladbarkeit mit Signalmolekülen. Ein weiterer Vorteil liegt in der stabilen Anbindung der Matrix an das Substrat. Hierfür wird durch die kovalente Anbringung beziehungsweise Nutzung des Haftvermittlers eine auch unter Scherbelastungen delaminationsstabile Kopplung der Materialien gewährleistet.

Die zuvor aufgezeigten Eigenschaften werden durch das mehrstufige Immobilisierungs- und Aufreinigungsverfahren erreicht. Im ersten Verfahrensschritt a) wird auf TCP oder anderen Zellkulturträgern, zum Beispiel Deckgläsern, sogenannten mikrostrukturierten Kunststoffoberflächen oder Zellkulturträgern mit sogenannten 3D-Strukturen, insbesondere poröse Materialien oder Gewebesheets, die Haftvermittlerschicht kovalent gebunden. Als Haftvermittlerschicht zur Stabilisierung der von den Zellen sezernierten extrazellulären Matrix (EZM) sind Carbonsäuregruppen tragende Polymere vorgesehen. In einer besonders vorteilhaften Ausführungsform des erfindungsgemäßen Verfahrens sind als Haftvermittlerschicht zur Stabilisierung der von den Zellen sezernierten extrazellulären Matrix (EZM) Maleinsäureanhydrid-Copolymere vorgesehen. Dabei wird bevorzugt ein als Haftvermittlerschicht verwendetes Maleinsäureanhydrid-Copolymer aus einer Gruppe ausgewählt, die die Copolymere Poly(octadecen-*alt-*maleinsäureanhydrid) (POMA), Poly(propen-*alt*-maleinsäureanhydrid) (PPMA), Poly(styren-*alt*-maleinsäureanhydrid) (PSMA) und Poly(ethylen-*alt-*maleinsäureanhydrid) (PEMA) umfasst. Ebenso können als Haftvermittlerschicht zur Stabilisierung der von den Zellen sezernierten extrazellulären Matrix (EZM) Polyacrylate verwendet werden.

Auf dem Haftvermittler werden im Anschluss daran im zweiten Verfahrensschritt b) zuerst eine Schicht eines EZM-Proteins kovalent gebunden, auf welcher dann Zellen eines gewünschten Organismus kultiviert und somit die von ihnen sezernierte Matrix abgeschieden und an die Haftvermittlerschicht angebunden wird. Gemäß einer Ausführungsform des Verfahrens werden dabei als zu kultivierende Zellen mesenchymale Stammzellen verwendet. Ebenso können in Verfahrensschritt b) auch fibroblastenartige Zelllinien kultiviert werden.

Im Anschluss an Verfahrensschritt b) wird in einem dritten Verfahrensschritt c) ein Dezellularisierungsprotokoll angewandt, um die matrixsezernierenden Zellen von der Oberfläche zu entfernen und gleichzeitig die Struktur und Funktionalität der darunterliegenden, mit dem Haftvermittler verbundenen Matrix zu erhalten. Das heißt, es erfolgt zunächst die Entfernung/Auflösung/Zersetzung der vorhanden Zellschicht, zum Beispiel durch Auflösen der Zellmembran. Dabei werden die funktionellen Bestandteile der extrazellulären Matrix, die durch einige der für die Dezellularisierung benötigten Reagenzien auch angegriffen werden können, erhalten.

Es findet dabei in Verfahrensschritt c) eine Dezellularisierungslösung Anwendung, die sich aus 5 Prozent Triton x-100 und 20 Millimol (mM) Ammoniumhydroxid (NH₄OH) pro Liter in phosphatgepufferter Salzlösung (PBS) zusammensetzt. In dieser Dezellularisierungslösung werden die mesenchymalen Stammzellenschichten (MSC) und extrazellulären Matrix-(EZM)-Schichten in einem Temperaturbereich von 35 °C bis 39 °C inkubiert. Vorzugsweise beträgt die Temperatur 37 °C und die Inkubationszeit mindestens zwei Minuten. Um die so gewonnenen Matrix-Schichten zu reinigen und vom Dezellularisierungspuffer zu befreien, schließt sich ein Waschprozess, vorzugsweise in drei Waschschritten mit phosphatgepufferter Salzlösung (PBS), an.

Jeder einzelne Verfahrensschritt ist durch ein hohes Maß an Flexibilität gekennzeichnet. Die Art der Ankopplung der Haftvermittlerschicht, der Haftvermittler selbst, die Art der Zellen oder Organkulturen, das Dezellularisierungsprotokoll und die Art der Refunktionalisierung, insbesondere Wachstumsfaktoren und Adhäsionsequenzen, sind austauschbar beziehungsweise an einzelne Fragestellungen gezielt anpassbar. Im Verfahrensschritt b), der Matrixsezernierung, können zudem gezielt stimulierende Bedingungen, zum Beispiel im Hinblick auf Zelldifferenzierung, eingestellt und somit auch die Matrixsezernierung beeinflusst werden. Durch diesen modularen Charakter kann die extrazelluläre Matrix-Beschichtung an eine Vielzahl von Anwendungen angepasst und für bestimmte zellbiologische Fragestellungen optimiert werden. Weiterhin wird durch den Einsatz der kovalent gekoppelten Haftvermittlerschicht ein deutlicher Stabilitätsgewinn erzielt, der einerseits das Handling der Matrixkultur allgemein erleichtert. Für die nachfolgende Kultivierung der Zielzellen auf den immobilisierten Matrix-Schichten können zudem andererseits eine deutlich größere Bandbreite an Verfahrensschritten angewandt werden, es sind zum Beispiel auch Kultivierungsbedingungen unter Scherstress möglich. Dies hat wiederum eine deutliche Erweiterung des Anwendungsspektrums derartiger Beschichtungen gegenüber bestehenden Einzelkomponenten-Matrixbeschichtungen zur Folge. Andererseits können durch die stabile Anbindung auch eine Vielzahl unterschiedlicher Dezellularisierungsprotokolle angewandt werden, womit auch von diesem Parameter her die resultierenden Matrixeigenschaften besser einstellbar und somit an einzelne Fragestellung besser anpassbar sind.

Als weiterer Vorteil ist die - auch aufgrund der so unterschiedlichen Aufreinigungsprotokolle gezielt variierbare - Beladbarkeit/Funktionalisierbarkeit mit Signalmolekülen zu nennen. Gemäß der Erfindung erfolgt daher in einem zusätzlichen Verfahrensschritt d) eine Funktionalisierung der nach den Verfahrensschritten a) bis c) aufbereiteten extrazellulären Matrices mit Wachstumsfaktoren oder anderen Signalmolekülen. Dadurch wird gerade gegenüber Verfahren zur Immobilisierung von Einzelkomponenten eine deutliche Verbesserung in Bezug auf die Regulation von Zellschicksalsentscheidungen erreicht.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Fig. 1:**: **(A)** eine schematische Darstellung der durch mesenchymale Stammzellen generierten extrazellulären Matrix und
**(B)** ein Verfahrensschema zur Herstellung der kovalenten Anbindung von Fibronektin an einem mit Poly(octadecen-*alt-*maleinsäureanhydrid) beschichteten Glasdeckgläschen;
- **Fig. 2:**: mikroskopische Aufnahmen von der Anbindung der extrazellulären Matrix an TCP-Oberflächen und an mit Poly(octadecen-*alt-*maleinsäureanhydrid)/Fibronektin vorbeschichteten Oberflächen des Zellkulturträgers;
- **Fig. 3:**: hellfeld- und rasterelektronenmikroskopische Aufnahmen unterschiedlicher Arten von durch mesenchymale Stammzellen generierter extrazellulärer Matrix und
- **Fig. 4:**: durch mesenchymale Stammzellen generierte extrazelluläre Matrices auf unterschiedlichen Haftvermittlern zur Stabilisierung der Matrix-Schichten.

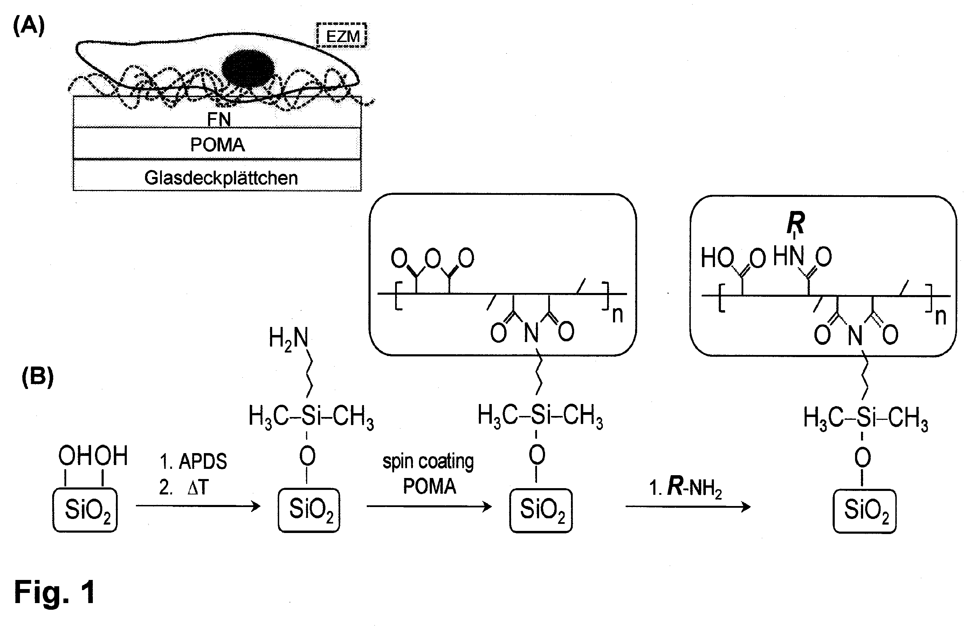

Das erste Ausführungsbeispiel I betrifft verschiedene, durch mesenchymale Stammzellen (MSC) generierte extrazelluläre Matrices (EZM) auf kovalent gebundenen Fibronektinschichten. Die Fig. 1 (A) zeigt dabei eine schematische Darstellung von mesenchymalen Stammzellen (MSC) generierter extrazellulärer Matrix (EZM) auf kovalent gebundenen, mit Fibronektin (FN) beschichteten Zellkulturträgern, welche in Form von Glasdeckgläsern vorliegen. Des Weiteren ist in Fig. 1 (B) ein Verfahrensschema zur Herstellung der kovalenten Anbindung von Fibronektin (FN, R) an dem mit Poly(octadecen-*alt*-maleinsäureanhydrid) (POMA) beschichteten Glasdeckgläschen dargestellt.

### Ausführungsbeispiel I, Verfahrensschritt a): Kovalente Anbindung des Haftvermittlers Fibronektin (FN, R) an mit Poly(octadecen-alt-maleinsäureanhydrid) (POMA) beschichtete Glasdeckgläser

Für die kovalente Anbindung von Fibronektin (FN, R) wurden polymerbehandelte Glasdeckgläschen verwendet, welche nach Pompe, T. et al. "Maleic anhydride copolymers - a versatile platform for molecular biosurface engineering". Biomacromolecules 4, 1072-1079 (2003**),** zunächst unter Wärmezufuhr mit 3-Aminopropyldimethylethoxysilan (APDS) und danach mit einer Lösung von Poly(octadecen-*alt*-maleinsäureanhydrid) (POMA) im Spin-Coating-Verfahren beschichtet worden waren. Diese reaktive Polymerschicht erlaubt die kovalente Anbindung von Aminogruppen (NH₂)-haltigen Molekülen über die vorhandenen Anhydridgruppen im Poly(octadecen-*alt*-maleinsäureanhydrid) (POMA), wie es in **Fig. 1 (B)** schematisch gezeigt wird. Die Kopplung von Aminogruppen (NH₂)-haltigem Fibronektin (FN, R), welches als extrazelluläres Matrixprotein (R) gemäß **Fig. 1 (B)** dient, erfolgte mit einer 50 µg/ml konzentrierten Lösung von humanem isoliertem Fibronektin (FN, R) in phosphatgepufferter Salzlösung (PBS). Für eine 6-Well-Zellkulturoberfläche von 9 cm² wurden 500 µl der Fibronektinlösung verwendet, um eine komplette Benetzung der Oberfläche zu gewährleisten. Nach einer Inkubationszeit von einer Stunde bei 37 °C wurde ungebundenes Fibronektin (FN, R) durch zwei Waschschritte mit 2 ml phosphatgepufferter Salzlösung (PBS) entfernt. Die auf diese Weise mit Fibronektin (FN) beschichteten Zellkulturträger (Glasdeckgläser) wurden unter 2 ml phosphatgepufferter Salzlösung (PBS) für kurze Zeit bei 4 °C und maximal sieben Tagen bis zur Weiterverarbeitung im nachfolgenden Verfahrensschritt b) gelagert.

### Ausführungsbeispiel I, Verfahrensschritt b): Matrixsezernierung durch Kultivierung von mesenchymalen Stammzellen (MSC) auf dem Haftvermittler

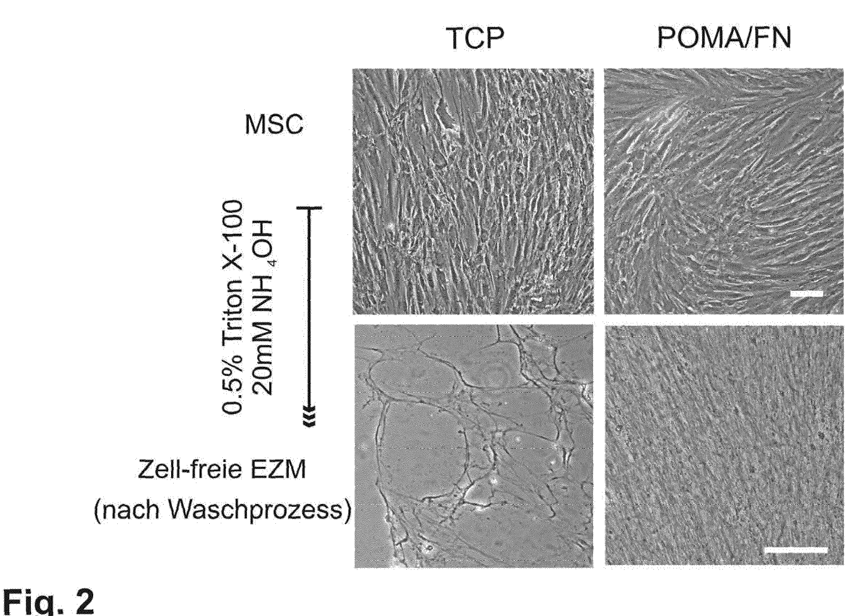

In diesem Verfahrensschritt wurden humane mesenchymale Stammzellen (MSC) zur Kultivierung und Matrixsezernierung verwendet. Isolierte primäre mesenchymale Stammzellen (MSC) aus humanem Knochenmark wurden durch das Universitätsklinikum Carl Gustav Carus in Dresden bereitgestellt. Zur Matrixsezernierung wurden nur frühe Passagen von mesenchymalen Stammzellen (MSC) verwendet (Passage 1 bis maximal 3). Die Zellen wurden bei einer Zelldichte von 10.000 Zellen/cm² auf die mit Fibronektin (FN, R) vorbeschichteten Zellkulturträger ausgesät. Mesenchymale Stammzellen (MSC) wurden in Dulbecco's Modified Eagle's Medium (DMEM) mit zehn Prozent Fetalem Kälberserum (FBS) kultiviert. Nach einer Anwachsphase von zwei bis drei Tagen erreichten die Zellen 80 bis 100 Prozent Konfluenz. Ab diesem Zeitpunkt wurde das Medium umgestellt, um die Zellen während der Matrix-Produktion mit unterschiedlichen Stimuli zu beeinflussen. Zur Anregung der Matrixsezernierung, insbesondere der Kollagensezernierung, wurden 50 µg/ml Ascorbinsäure zugesetzt, welches die Kollagensynthese stimuliert. Die hierbei entstehende Ascorbinsäure stimulierte extrazelluläre Matrix (EZM) wird als Ascorbinsäure-EZM beziehungsweise aaEZM bezeichnet. Weiterhin wurden die Zellen zur osteogenen Differenzierung stimuliert mittels Zugabe von 100 nM Dexamethasone, ß-Glycerolphosphat 10 mM und 2-Phospho-Ascorbinsäure. Die hieraus entstandene extrazelluläre Matrix (EZM) wird als osteoEZM bezeichnet. Ab dem Tag der Mediumumstellung wurden die Zellen für zehn Tage kultiviert und bekamen aller zwei Tage einen kompletten Mediumwechsel. Nach zehn Tagen wurde die Matrixsezernierung abgebrochen und die entstandenen Schichten der extrazellulären Matrix wurden dezellularisiert. Die **Fig. 2** zeigt in den oberen beiden Abbildungen jeweils die konfluente mesenchymale Stammzellenschicht vor der Dezellularisierung auf TCP-Oberflächen einerseits und auf mit Poly(octadecen-*alt-*maleinsäureanhydrid) (POMA)/Fibronektin (FN) vorbeschichteten Oberflächen des Zellkulturträgers andererseits.

### Ausführungsbeispiel I, Verfahrensschritt c): Dezellularisierung der zellsezernierten Schicht der extrazellulären Matrix (EZM) durch Triton X-100 und Ammoniumhydroxid NH₄OH)

Zur Vorbereitung der Dezellularisierung wurden die mesenchymalen Stammzellen-Kulturen mit 2 ml phosphatgepufferter Salzlösung (PBS) gewaschen. Die Dezellularisierungslösung setzte sich aus folgenden Bestandteilen zusammen: 5 Prozent Triton x-100 und 20 Millimol pro Liter Ammoniumhydroxid (NH₄OH) in phosphatgepufferter Salzlösung (PBS). Die Lösung wurde für jede Durchführung frisch hergestellt und auf 37 °C temperiert. Zu jeder 6-Well-Zellkulturoberfläche mit mesenchymalen Stammzellenschichten (MSC) beziehungsweise extrazellulären Matrix-(EZM)-Schichten wurde 1 ml Dezellularisierungspuffer gegeben und die mesenchymalen Stammzellenschichten (MSC) beziehungsweise extrazellulären Matrix-(EZM)-Schichten wurden anschließend bei 37 °C für fünf Minuten inkubiert. Der Dezellularsierungsprozess wurde lichtmikroskopisch kontrolliert. Alle zellulären Strukturen, zum Beispiel Zellkerne, müssen von der Oberfläche abschwimmen. Um die so gewonnenen Matrix-Schichten zu reinigen und vom Dezellularisierungspuffer zu befreien, schlossen sich drei Waschschritte mit phosphatgepufferter Salzlösung (PBS) an. Hierbei wurden jeweils 3 ml phosphatgepufferte Salzlösung (PBS) pro 6-Well zum Spülen verwendet. Jedoch durfte die Matrix dabei nie ganz trocken liegen. Nach Beendigung der Waschschritte wurde die Matrix in phosphatgepufferte Salzlösung (PBS) unter sterilen Bedingungen bei 4 °C für bis zu drei Monate gelagert. Die **Fig. 2** zeigt, dass extrazelluläre Matrix-(EZM)-Schichten, die auf Standard TCP-Zellkulturträgern generiert wurden, beim Waschprozess erhebliche Stabilitätsprobleme aufweisen. Im Gegensatz dazu konnten extrazelluläre Matrix-(EZM)-Schichten auf kovalent immobilisierten Fibronektin-(FN)-Oberflächen stabil gebunden und erhalten werden.

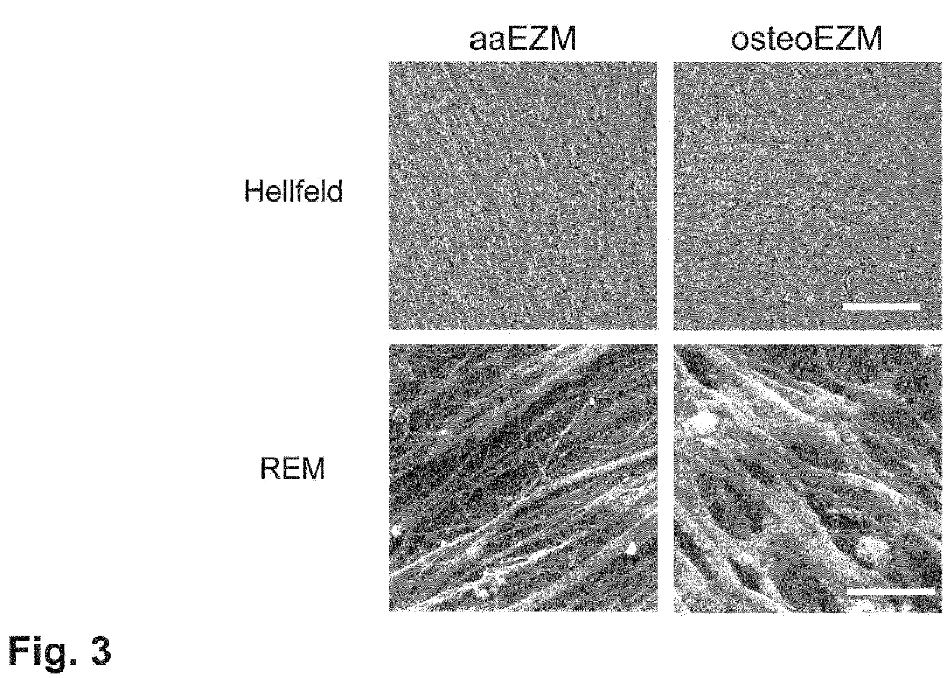

In **Fig. 3** wird das lichtmikroskopische und elektronenmikroskopische Erscheinungsbild der beiden oben genannten unterschiedlichen extrazellulären Matrix-Schichten (aaEZM-Schichten und osteoEZM-Schichten) nach deren Erzeugung durch die verschiedenen Stimuli während der Matrixsezernierung gezeigt. Die Messbalken entsprechen 100 µm bei hellfeldmikroskopischen Aufnahmen und 2 nm bei rasterelektronenmikroskopischen Aufnahmen (REM).

### Ausführungsbeispiel I, Verfahrensschritt d): Beladung der Matrices mit Wachstumsfaktoren/Cytokinen

Um die gewonnenen Matrices weiter zu variieren, wurde Stammzellenfaktor (SCF) adsorptiv an die Oberflächen gebunden. Dieses Cytokin spielt bei der Regulation von Hematopoietischen Stammzellen eine große Rolle, wie es auch aus Blank, U., Karlsson, G. & Karlsson, S. Signaling pathways governing stem cell fate. Blood (2007**),** bekannt ist. Eine Stammzellenfaktor-(SCF)-Lösung der Konzentration 100 ng/ml in phosphatgepufferter Salzlösung (PBS) wurde auf die Oberflächen der extrazellulären Matrix (EZM) aufgebracht. Je 6-Well-Kulturoberfläche mit extrazellulärer Matrixbeschichtung wurde 1 ml Stammzellenfaktor-(SCF)-Lösung für eine Stunde bei 4 °C inkubiert und im Anschluss einmalig mit 2 ml phosphatgepufferter Salzlösung (PBS) gewaschen. Die so funktionalisierten extrazellulären Matrix-(EZM)-Oberflächen wurden im Folgenden für Kulturexperimente mit Hematopoietischen Stammzellen eingesetzt.

Ein zweites Ausführungsbeispiel II betrifft die Stabilisierung einer durch mesenchymale Stammzellen (MSC) generierten extrazellulären Matrix (EZM) durch Maleinsäureanhydrid-Copolymere als Haftvermittler.

### Ausführungsbeispiel II, Verfahrensschritt a): Kovalente Bindung von Polymerdünnschichten an Glasträgern und zusätzliche kovalente Bindung von Fibronektin

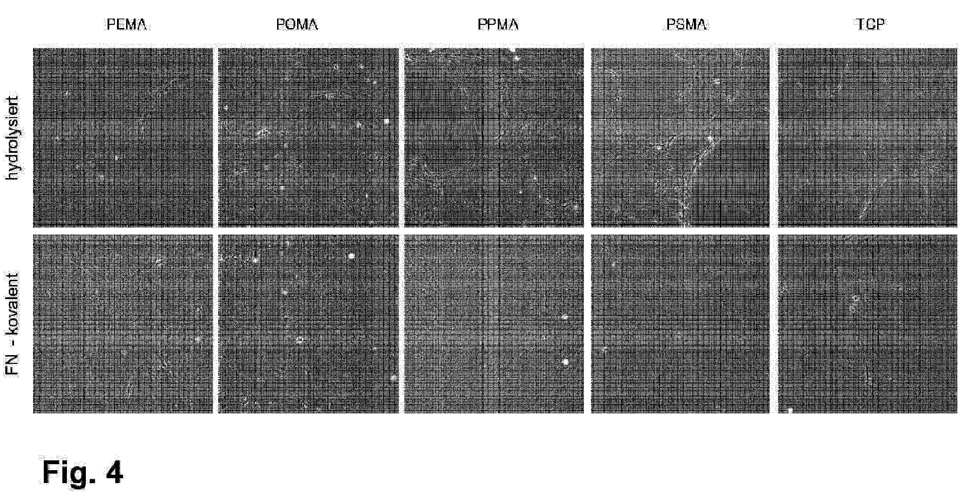

Weitere Haftvermittler zur Stabilisierung der zellsezernierten extrazellulären Matrix waren unterschiedliche Maleinsäureanhydrid-Copolymere: Poly(octadecen-*alt*-maleinsäureanhydrid) (POMA), Poly(propen-*alt-*maleinsäureanhydrid) (PPMA), Poly(styren-*alt*-maleinsäureanhydrid) (PSMA) und Poly(ethylen-*alt*-maleinsäureanhydrid) (PEMA). Es wurden Dünnschichtpräparationen dieser Polymere auf Glasoberflächen gemäß dem durch Pompe, T. et al. "Maleic anhydride copolymers - a versatile platform for molecular biosurface engineering". Biomacromolecules 4, 1072-1079 (2003**),** bekannten Verfahren generiert. Die **Fig. 4** zeigt mikroskopische Hellfeldaufnahmen der durch mesenchymale Stammzellen generierten extrazellulären Matrix auf den oben genannten unterschiedlichen, der Stabilisierung der Matrix-Schichten dienenden Haftvermittlern.

Die oben genannten erzeugten Polymeroberflächen unterschieden sich in ihren Eigenschaften hinsichtlich der Hydrophobizität. Weiterhin wurden diese Oberflächen entweder mit Fibronektin (FN, R) funktionalisiert, wie schon im ersten Ausführungsbeispiel I in Verfahrensschritt a) beschrieben, oder wurden mittels Wasser hydrolysiert, um kovalente Bindungen durch reaktive Seitengruppen zu unterbinden und allein Wechselwirkungen aufgrund der Hydrophobizität zu erhalten.

### Ausführungsbeispiel II, Verfahrensschritt b): Matrixsezernierung durch Kultivierung von mesenchymalen Stammzellen (MSC) auf den verschiedenen Haftvermittlern

Zur Matrix-Sezernierung wurden, wie schon im ersten Ausführungsbeispiel I beschrieben, mesenchymale Stammzellkulturen (MSC) verwendet. Im Unterschied zum ersten Ausführungsbeispiel I wurden jedoch keine weiteren Stimuli auf die Zellen ausgeübt. Die Kulturen wurden zehn Tage lang auf den verschiedenen, in Verfahrensschritt a) beschriebenen Oberflächen kultiviert.

### Ausführungsbeispiel II, Verfahrensschritt c): Dezellularisierung der zellsezernierten extrazellulären Matrix-(EZM)-Schicht durch Triton X-100 und 20 mM Ammoniumhydroxid (NH₄OH)

Die Dezellularisierung der extrazellulären Matrix-(EZM)-Schichten erfolgte identisch zu dem im ersten Ausführungsbeispiel I, Verfahrensschritt c), beschriebenen Protokoll. Die Effektivität der unterschiedlichen Oberflächen in Bezug auf die Anbindung der extrazellulären Matrix ist in **Fig. 4** dargestellt. Die verschiedenen hydrolysierten Formen der Maleinsäureanhydrid-Copolymere weisen eine Verbesserung der Stabilität der extrazellulären Matrix-(EZM)-Schichten auf. Besonders Poly(octadecen-*alt*-maleinsäureanhydrid) (POMA) zeigt durch hohe Hydrophobizität eine verbesserte Interaktion der sezernierten Matrix mit der Oberfläche. Wie die **Fig. 4** darüber hinaus verdeutlicht, kann durch kovalente Anbindung von Fibronektin (FN) an die verschiedenen Maleinsäureanhydrid-Copolymere die Stabilisierung der extrazellulären Matrix-(EZM)-Schichten noch verbessert werden.

### LISTE DER ABKÜRZUNGEN

- APDS: 3-Aminopropyldimethylethoxysilan
- DMEM: Dulbecco's Modified Eagle's Medium
- EZM: extrazelluläre Matrix
- aaEZM: extrazelluläre Matrix (bei einer durch Ascorbinsäure stimulierten Matrixsezernierung)
- osteoEZM: extrazelluläre Matrix (bei einer stimulierten Matrixsezenierung, die zu einer osteogenen Differenzierung führt)
- FBS: Fetales Kälberserum
- FN: Fibronektin
- LAS: lineares Alkylbenzolsulfonat
- MSC: mesenchymale Stammzellen
- PEMA: Poly(ethylen-*alt*-maleinsäureanhydrid)
- PBS: phosphatgepufferte Salzlösung
- POMA: Poly(octadecen-*alt*-maleinsäureanhydrid)
- PPMA: Poly(propen-*alt*-maleinsäureanhydrid)
- PSMA: Poly(styren-*alt*-maleinsäureanhydrid)
- R: extrazelluläres Matrixprotein, Fibronektin
- REM: Röntgenelektronenmikroskopische Aufnahme
- SCF: Stammzellenfaktor
- SDC: Natriumdeoxycholat
- SDS: Natriumdodecylsulfat
- TCP: Tissue Culture Plastics

## Patentansprüche

1. Verfahren zur Immobilisierung und Aufbereitung funktioneller Multikomponentenstrukturen der extrazellulären Matrix (EZM), umfassend die aufeinander folgenden Verfahrensschritte:
a) kovalente Bindung einer Haftvermittlerschicht aus Carbonsäuregruppen tragenden Polymeren, einschließlich Maleinsäureanhydrid-Copolymere und Polyacrylate, auf Zellkulturträgern;
b) Kultivierung von mesenchymalen Stammzellen (MSC) auf der Haftvermittlerschicht und somit Immobilisierung der von den Zellen sezernierten extrazellulären Matrix (EZM) durch Abscheidung und Anbindung an die Haftvermittlerschicht; wobei die Haftvermittlerschicht der Stabilisierung der von den Zellen sezernierten extrazellulären Matrix (EZM) dient, und
c) Anwendung eines Dezellularisierungsprotokolls, um die matrixsezernierenden Zellen von der Oberfläche zu entfernen und gleichzeitig die Struktur und Funktionalität der darunterliegenden, mit dem Haftvermittler verbundenen immobilisierten extrazellulären Matrix (EZM) zu erhalten; und
d) die Funktionalisierung der nach den Verfahrensschritten a) bis c) aufbereiteten extrazellulären Matrix (EZM) mit Signalmolekülen,
**dadurch gekennzeichnet, dass** in Verfahrensschritt c) die mesenchymalen Stammzellenschichten (MSC) und extrazellulären Matrix-(EZM)-Schichten in einer Dezellularisierungslösung, die sich aus 5 Prozent Triton x-100 und 20 mM Ammoniumhydroxid (NH4OH) in phosphatgepufferter Salzlösung (PBS) zusammensetzt, in einem Temperaturbereich von 35 °C bis 39 °C inkubiert und anschließend mit phosphatgepufferter Salzlösung (PBS) gewaschen werden.

2. Verfahren nach Anspruch 1, **dadurch gekennzeichnet, dass** als Zellkulturträger sogenannte Tissue Culture Plastics (TCP) oder Deckgläser oder sogenannte mikrostrukturierte Kunststoffoberflächen oder sogenannte 3D-Strukturen, insbesondere poröse Materialien oder Gewebesheets, vorgesehen sind.

3. Verfahren nach Anspruch 1 oder 2, **dadurch gekennzeichnet, dass** ein als Haftvermittlerschicht verwendetes Maleinsäureanhydrid-Copolymer aus einer Gruppe ausgewählt wird, die Poly(octadecen-*alt*-maleinsäureanhydrid) (POMA), Poly(propen-*alt*-maleinsäureanhydrid) (PPMA), Poly(styren-*alt-*maleinsäureanhydrid) (PSMA) und Poly(ethylen-*alt*-maleinsäureanhydrid) (PEMA) umfasst.

4. Verfahren nach einem der Ansprüche 1 bis 3, **dadurch gekennzeichnet, dass** an eine Haftvermittlerschicht aus einem Maleinsäureanhydrid-Copolymer für eine verbesserte Stabilisierung der extrazellulären Matrix-(EZM)-Schichten Fibronektin kovalent angebunden ist.

5. Verfahren nach einem der Ansprüche 1 bis 4, **dadurch gekennzeichnet, dass** die Funktionalisierung durch Beladung der immobilisierten extrazellulären Matrix (EZM) mit Wachstumsfaktoren erfolgt.

## Claims

1. Method for immobilising and processing functional multi-component structures of the extracellular matrix (ECM), comprising the following successive method steps:
a) covalent bonding of an adhesion promoter layer composed of carboxylic acid-bearing polymers, including maleic anhydride copolymers and polyacrylates, to cell culture carriers;
b) cultivation of mesenchymal stem cells (MSC) on the adhesion promoter layer and subsequent immobilisation of the extracellular matrix (ECM) secreted by the cells through deposition and connection to the adhesion promoter layer; whereby the adhesion promoter layer serves to stabilise the extracellular matrix (ECM) secreted by the cells, and
c) application of a decellularisation protocol in order to remove the matrix-secreting cells from the surface whilst simultaneously maintaining the structure and functionality of the underlying, immobilised extracellular matrix (ECM) connected with the adhesion promoter; and
d) the functionalisation of the extracellular matrix (ECM) processed according to method steps a) to c) with signalling modules, **characterised in that** in method step c) the mesenchymal stem cell (MSC) layers and extracellular matrix (ECM) layers are incubated in a decellularisation solution, which is composed of 5 percent Triton x-100 and 20 mM ammonium hydroxide (NH40H) in a phosphate-buffered saline solution (PBS), in a temperature range of between 35°C to 39 °C and are then washed with a phosphate-buffered saline solution (PBS).

2. Method according to claim 1, **characterised in that** so-called tissue culture plastics (TCP) or cover glasses or so-called micro-structured plastic surfaces or so-called 3D structures, in particular porous materials or fabric sheets, are intended as cell culture carriers.

3. Method according to claim 1 or 2, **characterised in that** a maleic anhydride copolymer used as an adhesion promoter layer is selected from a group including poly(octadecene-alt-maleic anhydride) (POMA), poly(propene-alt-maleic anhydride) (PPMA), poly(styrene-alt-maleic anhydride) (PSMA) and poly(ethylene-alt-maleic anhydride) (PEMA).

4. Method according one of claims 1 to 3, **characterised in that** fibronectin is covalently bonded to an adhesion promoter layer composed of a maleic anhydride copolymer for increased stabilisation of the extracellular matrix (ECM) layers.

5. Method according to one of claims 1 to 4, **characterised in that** the functionalisation can be achieved through loading the immobilised extracellular matrix (ECM) with growth factors.

## Revendications

1. Procédé pour l'immobilisation et la préparation de structures de multicomposants fonctionnelles de matrice extracellulaire (MEC), comprenant les étapes suivantes :
a) liaison par covalence d'une couche de promoteur d'adhérence composée de polymères porteurs de groupes d'acide carboxylique, y compris de copolymères d'anhydride maléique et de polyacrylates, sur des supports de culture cellulaire ;
b) culture de cellules souches mésenchymateuses (CSM) sur la couche de promoteur d'adhérence et, de ce fait, immobilisation de la matrice extracellulaire (MEC) sécrétée par les cellules, par séparation et liaison à la couche de promoteur d'adhérence, la couche de promoteur d'adhérence servant à la stabilisation de la matrice extracellulaire (MEC) sécrétée par les cellules ; et
c) application d'un protocole de décellularisation pour éliminer de la surface les cellules sécrétant la matrice et, en même temps, pour obtenir la structure et la fonctionnalité de la matrice extracellulaire (MEC) immobilisée sous-jacente, liée au promoteur d'adhérence ; et
d) la fonctionnalisation de la matrice extracellulaire (MEC) préparée selon les étapes a) à c) avec des molécules de signalisation, **caractérisé en ce que** les couches de cellules souches mésenchymateuses (CSM) et les couches de matrice extracellulaire (MEC) sont, à l'étape c), incubées dans une solution de décellularisation, composée de 5 % de Triton X-100 et de 20 mM d'hydroxyde d'ammonium (NH4OH) dans une solution saline tamponnée au phosphate (SSTP), dans une plage de températures comprises entre 35 °C et 39 °C, puis lavées avec une solution saline tamponnée au phosphate (SSTP).

2. Procédé selon la revendication 1, **caractérisé en ce que** les « Tissue Culture Plastics (TCP) » ou lamelles couvre-objets ou les surfaces de matière plastique microstructurées ou structures 3D, en particulier les matières poreuses ou les feuilles de tissu, sont prévues comme supports de culture cellulaire.

3. Procédé selon la revendication 1 ou 2, **caractérisé en ce qu'**un copolymère d'anhydride maléique utilisé comme couche de promoteur d'adhérence est choisi dans un groupe composé de poly(octadécène-*alt*-anhydride maléique) (POMA), de poly(propène-*alt*-anhydride maléique) (PPMA), de poly(styrène-*alt*-anhydride maléique) (PSMA) et de poly(éthylène-*alt*-anhydride maléique) (PEMA).

4. Procédé selon l'une des revendications 1 à 3, **caractérisé en ce que** la fibronectine est liée par covalence à une couche de promoteur d'adhérence composée d'un copolymère d'anhydride maléique pour une meilleure stabilisation des couches de matrice extracellulaire (MEC).

5. Procédé selon l'une des revendications 1 à 4, **caractérisé en ce que** la fonctionnalisation se fait en chargeant la matrice extracellulaire (MEC) immobilisée de facteurs de croissance.
